# FASCICULE DE BREVET EUROPEEN

(11) **EP 3 947 328 B1**
(45) Date de publication et mention de la délivrance du brevet: **19.04.2023**
(21) Numéro de dépôt: 20714235.7
(22) Date de dépôt: 01.04.2020
(51) Int. Cl.: C07C 17/20, C07C 17/395, C07C 21/18

(54) **PROCÉDÉ DE PURIFICATION DU 1-CHLORO-3,3,3-TRIFLUOROPROPÈNE**
VERFAHREN ZUR REINIGUNG VON 1-CHLOR-3,3,3-TRIFLUORPROPEN
METHOD FOR PURIFYING 1-CHLORO-3,3,3-TRIFLUOROPROPENE

(30) Priorité: 03.04.2019 FR 1903536
(43) Date de publication de la demande: 09.02.2022
(73) Titulaire: ARKEMA FRANCE, 92700 Colombes (FR)
(72) Inventeur: BOUSSARIE, Emmanuel, 69491 PIERRE-BENITE CEDEX (FR); HISLER, Kevin, 69491 PIERRE-BENITE CEDEX (FR); PIGAMO, Anne, 69491 PIERRE-BENITE CEDEX (FR); COLLIER, Bertrand, 69491 PIERRE-BENITE CEDEX (FR)
(74) Mandataire: Arkema Patent
(86) Numéro de dépôt international: PCT/EP2020/059231
(87) Numéro de publication internationale: WO 2020/201340

(56) Documents cités:
- EP-A1- 1 130 010
- WO-A1-2016/148957
- WO-A1-2020/101824
- WO-A1-2020/101825
- FR-A- 926 601
- US-A1- 2014 275 662

## Description

### Domaine technique de l'invention

La présente invention concerne un procédé de purification d'hydrochlorofluorooléfines. En particulier, la présente invention concerne un procédé de purification du 1-chloro-3,3,3-trifluoropropène.

### Arrière-plan technologique de l'invention

Le 3,3,3-trifluoro-1-chloropropène ou encore 1-chloro-3,3,3-trifluoropropène (HCFO-1233zd) existe sous forme de deux isomères : l'isomère cis, à savoir le Z-3,3,3-trifluoro-1-chloropropène (HCFO-1233zdZ), et l'isomère trans, à savoir le E-3,3,3-trifluoro-1-chloropropène (HCFO-1233zdE). Ils ont des points d'ébullition différents, respectivement de 18,5°C pour le composé trans et de 39,5°C pour le composé cis.

Les fluides à base de E-3,3,3-trifluoro-1-chloropropène (HCFO-1233zdE) ont trouvé de nombreuses applications dans des domaines industriels variés, notamment en tant que fluides de transfert de chaleur, propulseurs, agents moussants, agents gonflants, diélectriques gazeux, milieux de polymérisation ou monomères, fluides supports, agents abrasifs, agents de séchage et fluides pour unité de production d'énergie.

La fabrication du HCFO-1233zdE s'accompagne d'une multitude de sous-produits, ayant un point d'ébullition proche du HCFO-1233zdE. Cela conduit à des étapes de purification assez complexes et coûteuses. Les difficultés rencontrées au cours de la purification du HCFO-1233zdE impliquent généralement une perte conséquente en produit recherché. De plus, les sous-produits peuvent former des compositions azéotropiques avec le HCFO-1233zdE, rendant très difficile voire impossible la séparation par distillation simple. Les étapes de purification incluent notamment une étape de neutralisation du produit brut en sortie du réacteur pour éliminer les traces résiduelles d'acides, i.e. HCl et HF. Cette étape de neutralisation s'effectue généralement par l'intermédiaire d'une solution basique.

On connait notamment par WO2015/167784 un procédé de séparation du HCFO-1233zd et de l'HF par une série d'étapes incluant par exemple une distillation pour éliminer l'HCl en tête de colonne de distillation, le refroidissement du flux en pied de colonne pour obtenir un mélange bi-phasique, la séparation des deux phases et le traitement d'une de celle-ci par un adsorbant pouvant être un adsorbant liquide (eau, NaOH ou KOH).

On connait aussi par WO2016/148957 un procédé de purification du HCFO-1233zdE comprenant une étape de lavage, une étape de condensation et de séparation de phase et, enfin une étape de séchage. On connait également par WO2014/010530, WO2014/189674, WO2014/099464 un procédé de préparation du HCFO-1233zd comprenant une étape de purification telle qu'un lavage à l'eau ou un lavage à l'aide d'une solution basique.

Lors de la purification du HCFO-1233zd, le traitement mis en oeuvre pour éliminer des impuretés acides telles que HF ou HCl peut contribuer à la formation de certaines impuretés.

Ainsi, de nouvelles conditions de neutralisation des impuretés acides doivent être mises en oeuvre pour minimiser la dégradation du HCFO-1233zd. Il existe un besoin pour un procédé efficace de purification du trans-1-chloro-3,3,3-trifluoropropène minimisant la production de sous-produits ou autres impuretés issues d'une réaction de déshydrochloration.

### Résumé de l'invention

Selon un premier aspect, la présente invention concerne un procédé de purification du 1-chloro-3,3,3-trifluoropropène comprenant les étapes de :
a) fourniture d'un courant comprenant 1-chloro-3,3,3-trifluoropropène et au moins un composé de formule HX dans laquelle X est F ou CI ;
b) mise en contact du courant de l'étape a) avec une solution **A** comprenant au moins un hydroxyde alcalin ou un hydroxyde d'alcalino-terreux et au moins un sel de sulfite de formule Yⁿ⁺ₘSO₃ dans laquelle Y est un métal alcalin ou alcalino-terreux, n = 1 ou 2, et m = 2 lorsque n = 1 ou m = 1 lorsque n = 2 ; pour former un courant neutralisé **A1** comprenant 1-chloro-3,3,3-trifluoropropène.

Selon un mode de réalisation préféré, ledit au moins un sel de sulfite est sélectionné parmi le groupe consistant en Na₂SO₃, K₂SO₃, Li₂SO₃, CaSO₃ et MgSO₃ ou un mélange de ceux-ci.

Selon un mode de réalisation préféré, ledit au moins un sel de sulfite est Na₂SO₃ ou K₂SO₃ ou un mélange des deux.

Selon un mode de réalisation préféré, ladite solution **A** comprend au moins un hydroxyde alcalin, de préférence NaOH.

Selon un mode de réalisation préféré, ladite solution **A** de l'étape b) comprend un mélange de NaOH et Na₂SO₃.

Selon un mode de réalisation préféré, ladite solution **A** de l'étape b) est une solution aqueuse. Selon un mode de réalisation préféré, la teneur en ledit au moins un sel de sulfite est comprise entre 2 et 25% en poids sur base du poids total de ladite solution **A.**

Selon un mode de réalisation préféré, l'étape b) est mise en oeuvre à une température de 10°C à 70°C.

Selon un mode de réalisation préféré, la teneur en hydroxyde alcalin ou en hydroxyde d'alcalino-terreux est comprise entre 2 et 25% en poids sur base du poids total de ladite solution **A.**

Selon un mode de réalisation préféré, l'étape b) est mise en oeuvre avec une solution aqueuse **A** comprenant de 3 à 10% de Na₂SO₃, de 3 à 10% de NaOH ; et à une température de 20°C à 60°C Selon un mode de réalisation préféré, dans le courant fourni à l'étape a), le ratio molaire entre l'isomère trans et l'isomère cis du 1-chloro-3,3,3-trifluoropropène est de 2:1 à 50:1, de préférence de 5 :1 à 50 :1, en particulier de 9 :1 à 50 :1.

De préférence, le présent procédé se rapporte à un procédé de purification du trans-1-chloro-3,3,3-trifluoropropène comprenant les étapes de :
a) fourniture d'un courant comprenant trans-1-chloro-3,3,3-trifluoropropène et au moins un composé de formule HX dans laquelle X est F ou CI ;
b) mise en contact du courant de l'étape a) avec une solution **A** comprenant au moins un hydroxyde alcalin ou un hydroxyde d'alcalino-terreux et au moins un sel de sulfite de formule Yⁿ⁺ₘSO₃ dans laquelle Y est un métal alcalin ou alcalino-terreux, n = 1 ou 2, et m = 2 lorsque n = 1 ou m = 1 lorsque n = 2 ; pour former un courant neutralisé **A1** comprenant trans-1-chloro-3,3,3-trifluoropropène.

Selon un second aspect, le présente invention fournit un procédé de production du 1-chloro-3,3,3-trifluoropropène comprenant les étapes de :
i) mise en contact, dans un réacteur, entre l'acide fluorhydrique (HF) et une composition de départ comprenant au moins un composé chloré sélectionné parmi le groupe consistant en 1,1,3,3-tetrachloropropene, 1,3,3,3-tetrachloropropene et un mélange des deux, pour obtenir un courant **B** comprenant 1-chloro-3,3,3-trifluoropropène, HF et/ou HCl ;
ii) mise en oeuvre du procédé de purification de 1-chloro-3,3,3-trifluoropropène selon la présente invention à partir d'un courant comprenant 1-chloro-3,3,3-trifluoropropène, HF et/ou HCl.

### Description détaillée de la présente invention

Selon un premier aspect, la présente invention concerne un procédé de purification du 1-chloro-3,3,3-trifluoropropène, de préférence du trans-1-chloro-3,3,3-trifluoropropène.

Selon un mode de réalisation préféré, ledit procédé de purification comprend :
a) fourniture d'un courant comprenant 1-chloro-3,3,3-trifluoropropène et au moins un composé de formule HX dans laquelle X est F ou CI ;
b) mise en contact du courant de l'étape a) avec une solution **A** comprenant au moins un hydroxyde alcalin ou un hydroxyde d'alcalino-terreux et au moins un sel de sulfite de formule Yⁿ⁺ₘSO₃ dans laquelle Y est un métal alcalin ou alcalino-terreux, n = 1 ou 2, et m = 2 lorsque n = 1 ou m = 1 lorsque n = 2 ; pour former un courant neutralisé **A1** comprenant 1-chloro-3,3,3-trifluoropropène.

Selon un mode de réalisation particulier, ledit procédé de purification comprend :
a) fourniture d'un courant comprenant trans-1-chloro-3,3,3-trifluoropropène et au moins un composé de formule HX dans laquelle X est F ou CI ;
b) mise en contact du courant de l'étape a) avec une solution **A** comprenant au moins un hydroxyde alcalin ou un hydroxyde d'alcalino-terreux et au moins un sel de sulfite de formule Yⁿ⁺ₘSO₃ dans laquelle Y est un métal alcalin ou alcalino-terreux, n = 1 ou 2, et m = 2 lorsque n = 1 ou m = 1 lorsque n = 2 ; pour former un courant neutralisé **A1** comprenant trans-1-chloro-3,3,3-trifluoropropène.

Le demandeur a notamment observé que l'utilisation d'un sel de sulfite de formule Yⁿ⁺ₘSO₃ tel que défini ci-dessus permettait de limiter la formation d'impuretés lors de l'étape b). Ces impuretés peuvent être issues de la dégradation du 1-chloro-3,3,3-trifluoropropène, de l'isomère cis et/ou de l'isomère trans, ou éventuellement d'autres composés présents dans le flux de départ, au cours de l'étape b). Cette étape permet la neutralisation du ou des composés de formule HX tel que défini ci-dessus. Le présent procédé de purification permet ainsi de récupérer un courant **A1** comprenant 1-chloro-3,3,3-trifluoropropène, de préférence trans-1-chloro-3,3,3-trifluoropropène, dans lequel la teneur en composé de formule HX est fortement diminuée tout en limitant la formation d'impuretés additionnelles. Parmi les impuretés additionnelles, on peut citer notamment la trifluoropropyne dont la présence est désavantageuse. De préférence, le composé HX est HCl ou HF ou un mélange de ceux-ci.

Le courant fourni à l'étape a) peut comprendre un mélange d'isomères cis/trans du 1-chloro-3,3,3-trifluoropropène. De préférence, dans le courant fourni à l'étape a), le ratio molaire entre l'isomère trans et cis du 1-chloro-3,3,3-trifluoropropène est de 2 :1 à 50 :1, de préférence de 5:1 à 50:1, en particulier de 9:1 à 50:1. En outre, le courant fourni à l'étape a) peut éventuellement comprendre 1,3,3,3-tétrafluoropropène et 1,1,1,3,3-pentafluoropropane, dans des teneurs explicitées ci-dessous dans la présente demande en relation avec le courant **B.**

De préférence, ledit au moins un sel de sulfite de formule Yⁿ⁺ₘSO₃ est sélectionné parmi le groupe consistant en Na₂SO₃, K₂SO₃, Li₂SO₃, CaSO₃ et MgSO₃ ou un mélange de ceux-ci. Plus préférentiellement, ledit au moins un sel de sulfite de formule Yⁿ⁺ₘSO₃ est Na₂SO₃ ou K₂SO₃ ou un mélange de ceux-ci. En particulier, ledit au moins un sel de sulfite de formule Yⁿ⁺ₘSO₃ est Na₂SO₃.

De préférence, la teneur dans ledit au moins un sel de sulfite dans ladite solution **A** est comprise entre 1% et 50% en poids, plus préférentiellement entre 2% et 40% en poids, en particulier entre 2% et 30%, plus particulièrement entre 2% et 25% en poids sur base du poids total de ladite solution **A.**

Comme mentionné ci-dessus, ladite solution **A** de l'étape b) comprend au moins un hydroxyde alcalin ou un hydroxyde d'alcalino-terreux, ou un mélange des deux, en sus dudit sel de formule Yⁿ⁺ₘSO₃ tel que défini ci-dessus. L'hydroxyde alcalin peut être NaOH ou KOH. L'hydroxyde alcalino-terreux peut être Ca(OH)₂ ou Mg(OH)₂. De préférence, la teneur dudit au moins un hydroxyde alcalin ou alcalino-terreux dans ladite solution **A** est comprise entre 1% et 50% en poids, plus préférentiellement entre 2% et 40% en poids, en particulier entre 2% et 30%, plus particulièrement entre 2% et 25% en poids sur base du poids total de ladite solution **A.**

De préférence, ladite solution **A** de l'étape b) est une solution comprenant un sel de formule Yⁿ⁺ₘSO₃ et NaOH ou un sel de formule Yⁿ⁺ₘSO₃ et KOH ou un sel de formule Yⁿ⁺ₘSO₃, NaOH et KOH. En particulier, ladite solution **A** de l'étape b) est une solution comprenant Na₂SO₃ et NaOH ou Na₂SO₃ et KOH ou Na₂SO₃, NaOH et KOH.

De manière privilégiée, ladite solution **A** de l'étape b) est une solution aqueuse comprenant un sel de formule Yⁿ⁺ₘSO₃ et NaOH ou un sel de formule Yⁿ⁺ₘSO₃ et KOH ou un sel de formule Yⁿ⁺ₘSO₃, NaOH et KOH. En particulier, ladite solution **A** de l'étape b) est une solution aqueuse comprenant Na₂SO₃ et NaOH ou Na₂SO₃ et KOH ou Na₂SO₃, NaOH et KOH.

De préférence, ladite solution **A** de l'étape b) comprend entre 1% et 50% en poids, plus préférentiellement entre 2% et 40% en poids, en particulier entre 2% et 30%, plus particulièrement entre 2% et 25% d'un sel de formule Yⁿ⁺ₘSO₃ et entre 1% et 50% en poids, plus préférentiellement entre 2% et 40% en poids, en particulier entre 2% et 30%, plus particulièrement entre 2% et 25% de NaOH. Selon un autre mode préférentiel, ladite solution **A** de l'étape b) comprend entre 1% et 50% en poids, plus préférentiellement entre 2% et 40% en poids, en particulier entre 2% et 30%, plus particulièrement entre 2% et 25% d'un sel de formule Yⁿ⁺ₘSO₃ et entre 1% et 50% en poids, plus préférentiellement entre 2% et 40% en poids, en particulier entre 2% et 30%, plus particulièrement entre 2% et 25% de KOH. Selon un autre mode préférentiel, ladite solution **A** de l'étape b) comprend entre 1% et 50% en poids, plus préférentiellement entre 2% et 40% en poids, en particulier entre 2% et 30%, plus particulièrement entre 2% et 25% d'un sel de formule Yⁿ⁺ₘSO₃ ; entre 1% et 50% en poids, plus préférentiellement entre 2% et 40% en poids, en particulier entre 2% et 30%, plus particulièrement entre 2% et 25% de NaOH et entre 1% et 50% en poids, plus préférentiellement entre 2% et 40% en poids, en particulier entre 2% et 30%, plus particulièrement entre 2% et 25% de KOH.

De préférence, ladite solution **A** de l'étape b) est une solution aqueuse comprenant entre 1% et 50% en poids, plus préférentiellement entre 2% et 40% en poids, en particulier entre 2% et 30%, plus particulièrement entre 2% et 25% d'un sel de formule Yⁿ⁺ₘSO₃ et entre 1% et 50% en poids, plus préférentiellement entre 2% et 40% en poids, en particulier entre 2% et 30%, plus particulièrement entre 2% et 25% de NaOH. Selon un autre mode préférentiel, ladite solution **A** de l'étape b) est une solution aqueuse comprenant entre 1% et 50% en poids, plus préférentiellement entre 2% et 40% en poids, en particulier entre 2% et 30%, plus particulièrement entre 2% et 25% d'un sel de formule Yⁿ⁺ₘSO₃ et entre 1% et 50% en poids, plus préférentiellement entre 2% et 40% en poids, en particulier entre 2% et 30%, plus particulièrement entre 2% et 25% de KOH. Selon un autre mode préférentiel, ladite solution **A** de l'étape b) est une solution aqueuse comprenant entre 1% et 50% en poids, plus préférentiellement entre 2% et 40% en poids, en particulier entre 2% et 30%, plus particulièrement entre 2% et 25% d'un sel de formule Yⁿ⁺ₘSO₃ ; entre 1% et 50% en poids, plus préférentiellement entre 2% et 40% en poids, en particulier entre 2% et 30%, plus particulièrement entre 2% et 25% de NaOH et entre 1% et 50% en poids, plus préférentiellement entre 2% et 40% en poids, en particulier entre 2% et 30%, plus particulièrement entre 2% et 25% de KOH.

De préférence, ladite solution **A** de l'étape b) comprend entre 1% et 50% en poids, plus préférentiellement entre 2% et 40% en poids, en particulier entre 2% et 30%, plus particulièrement entre 2% et 25% de Na₂SO₃, et entre 1% et 50% en poids, plus préférentiellement entre 2% et 40% en poids, en particulier entre 2% et 30%, plus particulièrement entre 2% et 25% de NaOH. Selon un autre mode préférentiel, ladite solution **A** de l'étape b) comprend entre 1% et 50% en poids, plus préférentiellement entre 2% et 40% en poids, en particulier entre 2% et 30%, plus particulièrement entre 2% et 25% de Na₂SO₃ et entre 1% et 50% en poids, plus préférentiellement entre 2% et 40% en poids, en particulier entre 2% et 30%, plus particulièrement entre 2% et 25% de KOH. Selon un autre mode préférentiel, ladite solution A de l'étape b) comprend entre 1% et 50% en poids, plus préférentiellement entre 2% et 40% en poids, en particulier entre 2% et 30%, plus particulièrement entre 2% et 25% de Na₂SO₃ ; entre 1% et 50% en poids, plus préférentiellement entre 2% et 40% en poids, en particulier entre 2% et 30%, plus particulièrement entre 2% et 25% de NaOH et entre 1% et 50% en poids, plus préférentiellement entre 2% et 40% en poids, en particulier entre 2% et 30%, plus particulièrement entre 2% et 25% de KOH.

De préférence, ladite solution A de l'étape b) est une solution aqueuse comprenant entre 1% et 50% en poids, plus préférentiellement entre 2% et 40% en poids, en particulier entre 2% et 30%, plus particulièrement entre 2% et 25% de Na₂SO₃, et entre 1% et 50% en poids, plus préférentiellement entre 2% et 40% en poids, en particulier entre 2% et 30%, plus particulièrement entre 2% et 25% de NaOH. Selon un autre mode préférentiel, ladite solution A de l'étape b) est une solution aqueuse comprenant entre 1% et 50% en poids, plus préférentiellement entre 2% et 40% en poids, en particulier entre 2% et 30%, plus particulièrement entre 2% et 25% de Na₂SO₃ et entre 1% et 50% en poids, plus préférentiellement entre 2% et 40% en poids, en particulier entre 2% et 30%, plus particulièrement entre 2% et 25% de KOH. Selon un autre mode préférentiel, ladite solution A de l'étape b) est une solution aqueuse comprenant entre 1% et 50% en poids, plus préférentiellement entre 2% et 40% en poids, en particulier entre 2% et 30%, plus particulièrement entre 2% et 25% de Na₂SO₃ ; entre 1% et 50% en poids, plus préférentiellement entre 2% et 40% en poids, en particulier entre 2% et 30%, plus particulièrement entre 2% et 25% de NaOH et entre 1% et 50% en poids, plus préférentiellement entre 2% et 40% en poids, en particulier entre 2% et 30%, plus particulièrement entre 2% et 25% de KOH.

De préférence, l'étape b) du présent procédé est mis en oeuvre à une température de 10°C à 90°C, avantageusement de 10°C à 80°C, de préférence de 10°C à 70°C, en particulier de 10°C à 60°C.

Ainsi, selon un mode de réalisation préféré, l'étape b) est mise en oeuvre avec une solution A comprenant entre 1% et 50% en poids, plus préférentiellement entre 2% et 40% en poids, en particulier entre 2% et 30%, plus particulièrement entre 2% et 25% d'un sel de formule Yⁿ⁺ₘSO₃ et entre 1% et 50% en poids, plus préférentiellement entre 2% et 40% en poids, en particulier entre 2% et 30%, plus particulièrement entre 2% et 25% de NaOH ; à une température de 10°C à 90°C, avantageusement de 10°C à 80°C, de préférence de 10°C à 70°C, en particulier de 10°C à 60°C. Selon un mode de réalisation particulier, l'étape b) est mise en oeuvre avec une solution A comprenant entre 1% et 50% en poids, plus préférentiellement entre 2% et 40% en poids, en particulier entre 2% et 30%, plus particulièrement entre 2% et 25% d'un sel de formule Na₂SO₃ et entre 1% et 50% en poids, plus préférentiellement entre 2% et 40% en poids, en particulier entre 2% et 30%, plus particulièrement entre 2% et 25% de NaOH ; à une température de 10°C à 90°C, avantageusement de 10°C à 80°C, de préférence de 10°C à 70°C, en particulier de 10°C à 60°C.

Selon un autre mode de réalisation préféré, l'étape b) est mise en oeuvre avec une solution **A** comprenant entre 1% et 50% en poids, plus préférentiellement entre 2% et 40% en poids, en particulier entre 2% et 30%, plus particulièrement entre 2% et 25% d'un sel de formule Yⁿ⁺ₘSO₃ et entre 1% et 50% en poids, plus préférentiellement entre 2% et 40% en poids, en particulier entre 2% et 30%, plus particulièrement entre 2% et 25% de KOH ; à une température de 10°C à 90°C, avantageusement de 10°C à 80°C, de préférence de 10°C à 70°C, en particulier de 10°C à 60°C. Selon un mode de réalisation particulier, l'étape b) est mise en oeuvre avec une solution **A** comprenant entre 1% et 50% en poids, plus préférentiellement entre 2% et 40% en poids, en particulier entre 2% et 30%, plus particulièrement entre 2% et 25% d'un sel de formule Na₂SO₃ et entre 1% et 50% en poids, plus préférentiellement entre 2% et 40% en poids, en particulier entre 2% et 30%, plus particulièrement entre 2% et 25% de KOH ; à une température de 10°C à 90°C, avantageusement de 10°C à 80°C, de préférence de 10°C à 70°C, en particulier de 10°C à 60°C.

Selon un autre mode de réalisation préféré, l'étape b) est mise en oeuvre avec une solution **A** comprenant entre 1% et 50% en poids, plus préférentiellement entre 2% et 40% en poids, en particulier entre 2% et 30%, plus particulièrement entre 2% et 25% d'un sel de formule Yⁿ⁺ₘSO₃ ; entre 1% et 50% en poids, plus préférentiellement entre 2% et 40% en poids, en particulier entre 2% et 30%, plus particulièrement entre 2% et 25% de KOH et entre 1% et 50% en poids, plus préférentiellement entre 2% et 40% en poids, en particulier entre 2% et 30%, plus particulièrement entre 2% et 25% de NaOH ; à une température de 10°C à 90°C, avantageusement de 10°C à 80°C, de préférence de 10°C à 70°C, en particulier de 10°C à 60°C. Selon un mode de réalisation particulier, l'étape b) est mise en oeuvre avec une solution **A** comprenant entre 1% et 50% en poids, plus préférentiellement entre 2% et 40% en poids, en particulier entre 2% et 30%, plus particulièrement entre 2% et 25% d'un sel de formule Na₂SO₃ ; entre 1% et 50% en poids, plus préférentiellement entre 2% et 40% en poids, en particulier entre 2% et 30%, plus particulièrement entre 2% et 25% de KOH et entre 1% et 50% en poids, plus préférentiellement entre 2% et 40% en poids, en particulier entre 2% et 30%, plus particulièrement entre 2% et 25% de NaOH ; à une température de 10°C à 90°C, avantageusement de 10°C à 80°C, de préférence de 10°C à 70°C, en particulier de 10°C à 60°C. De préférence, selon un mode de réalisation préféré, l'étape b) est mise en oeuvre avec une solution aqueuse A comprenant entre 1% et 50% en poids, plus préférentiellement entre 2% et 40% en poids, en particulier entre 2% et 30%, plus particulièrement entre 2% et 25% d'un sel de formule Yⁿ⁺ₘSO₃ et entre 1% et 50% en poids, plus préférentiellement entre 2% et 40% en poids, en particulier entre 2% et 30%, plus particulièrement entre 2% et 25% de NaOH ; à une température de 10°C à 90°C, avantageusement de 10°C à 80°C, de préférence de 10°C à 70°C, en particulier de 10°C à 60°C. Selon un mode de réalisation particulier, l'étape b) est mise en oeuvre avec une solution aqueuse **A** comprenant entre 1% et 50% en poids, plus préférentiellement entre 2% et 40% en poids, en particulier entre 2% et 30%, plus particulièrement entre 2% et 25% d'un sel de formule Na₂SO₃ et entre 1% et 50% en poids, plus préférentiellement entre 2% et 40% en poids, en particulier entre 2% et 30%, plus particulièrement entre 2% et 25% de NaOH ; à une température de 10°C à 90°C, avantageusement de 10°C à 80°C, de préférence de 10°C à 70°C, en particulier de 10°C à 60°C. Selon un autre mode de réalisation préféré, l'étape b) est mise en oeuvre avec une solution aqueuse **A** comprenant entre 1% et 50% en poids, plus préférentiellement entre 2% et 40% en poids, en particulier entre 2% et 30%, plus particulièrement entre 2% et 25% d'un sel de formule Yⁿ⁺ₘSO₃ et entre 1% et 50% en poids, plus préférentiellement entre 2% et 40% en poids, en particulier entre 2% et 30%, plus particulièrement entre 2% et 25% de KOH ; à une température de 10°C à 90°C, avantageusement de 10°C à 80°C, de préférence de 10°C à 70°C, en particulier de 10°C à 60°C. Selon un mode de réalisation particulier, l'étape b) est mise en oeuvre avec une solution aqueuse **A** comprenant entre 1% et 50% en poids, plus préférentiellement entre 2% et 40% en poids, en particulier entre 2% et 30%, plus particulièrement entre 2% et 25% d'un sel de formule Na₂SO₃ et entre 1% et 50% en poids, plus préférentiellement entre 2% et 40% en poids, en particulier entre 2% et 30%, plus particulièrement entre 2% et 25% de KOH ; à une température de 10°C à 90°C, avantageusement de 10°C à 80°C, de préférence de 10°C à 70°C, en particulier de 10°C à 60°C.

Selon un autre mode de réalisation préféré, l'étape b) est mise en oeuvre avec une solution aqueuse **A** comprenant entre 1% et 50% en poids, plus préférentiellement entre 2% et 40% en poids, en particulier entre 2% et 30%, plus particulièrement entre 2% et 25% d'un sel de formule Yⁿ⁺ₘSO₃ ; entre 1% et 50% en poids, plus préférentiellement entre 2% et 40% en poids, en particulier entre 2% et 30%, plus particulièrement entre 2% et 25% de KOH et entre 1% et 50% en poids, plus préférentiellement entre 2% et 40% en poids, en particulier entre 2% et 30%, plus particulièrement entre 2% et 25% de NaOH ; à une température de 10°C à 90°C, avantageusement de 10°C à 80°C, de préférence de 10°C à 70°C, en particulier de 10°C à 60°C. Selon un mode de réalisation particulier, l'étape b) est mise en oeuvre avec une solution aqueuse **A** comprenant entre 1% et 50% en poids, plus préférentiellement entre 2% et 40% en poids, en particulier entre 2% et 30%, plus particulièrement entre 2% et 25% d'un sel de formule Na₂SO₃ ; entre 1% et 50% en poids, plus préférentiellement entre 2% et 40% en poids, en particulier entre 2% et 30%, plus particulièrement entre 2% et 25% de KOH et entre 1% et 50% en poids, plus préférentiellement entre 2% et 40% en poids, en particulier entre 2% et 30%, plus particulièrement entre 2% et 25% de NaOH ; à une température de 10°C à 90°C, avantageusement de 10°C à 80°C, de préférence de 10°C à 70°C, en particulier de 10°C à 60°C. Alternativement, ladite solution **A** peut être une solution organique contenant un solvant. Le solvant est défini comme un composé organique inerte dans lequel le 1-chloro-3,3,3-trifluoropropène est au moins partiellement soluble. Le solvant est de préférence sélectionné parmi le groupe consistant en hydrocarbures, éthers, alcools, halogénures d'alkyles, benzènes substitués ou non, nitrile d'alkyle, amides, sulfoxydes, sulfones, esters de phosphate et les mélanges de ceux-ci. De préférence, le solvant est sélectionné parmi éthers, alcools, halogénures d'alkyles, benzènes substitués ou non, nitrile d'alkyle, amides, sulfoxydes, sulfones, et les mélanges de ceux-ci. Les éthers incluent les éthers d'alkyle acyclique, les éthers cycliques, les éthers perfluorés, glyme, diglyme, triglyme, tetraglyme et les mélanges de ceux-ci. Les éthers d'alkyle acyclique incluent l'éther diméthylique, l'éther éthylique, l'éther de méthyle éthyle et les mélanges de ceux-ci. Les éthers cycliques incluent le 2-methyltetrahydrofurane, tetrahydrofurane, tétrahydropyrane, 1,4-dioxane et les mélanges de ceux-ci. Les éthers perfluorés incluent perfluoro-N-méthyl morpholine, perfluorotétrahydrofurane, et les mélanges de ceux-ci. Les alcools incluent les alcools d'alkyle, les glycols, glycérol, et les mélanges de ceux-ci. Les alkykes d'alcool incluent méthanol, éthanol, propanol, isopropanol, 2-méthyl-2-propanol, cyclohexanol, et les mélanges de ceux-ci. Les exemples de glycol incluent l'éthylène glycol, propylène glycol, diéthylène glycol et les mélanges de ceux-ci. Les benzènes substitués ou non incluent les alkylbenzènes, halobenzènes, benzonitrile phénol, anisole, biphényle, nitrobenzène et les mélanges de ceux-ci. Les alkylbenzènes incluent toluène, éthylbenzène, o-xylène, m-xylène, p-xylène, mésitylène, durène, 2-phénylhexane et les mélanges de ceux-ci. Les halobenzènes incluent fluorobenzène, chlorobenzène, 1,2-dichlorobenzène, 1,4-dichlorobenzène, et les mélanges de ceux-ci. Les halogénures d'alkyles incluent dichlorométhane, chloroforme, tétrachlorure de carbone, chloroéthane, 1,2-dichloroéthane et les mélanges de ceux-ci. Les nitrile d'alkyles incluent acétonitrile, butyronitrile, méthylglutaronitrile, adiponitrile et les mélanges de ceux-ci. Les amides incluent N,N-diméthyl formamide, N,N-diméthyl-acétamide, N-méthyl-2-pyrrolidone et les mélanges ceux-ci. Les sulfoxides incluent le diméthyle sulfoxyde. Les sulfones incluent le sulfolane.

Selon un second aspect de la présente invention, un procédé de production du 1-chloro-3,3,3-trifluoropropène est fourni ; de préférence, un procédé de production du trans-1-chloro-3,3,3-trifluoropropène.

Ledit procédé de production du 1-chloro-3,3,3-trifluoropropène comprenant les étapes de :
i) mise en contact, dans un réacteur, entre l'acide fluorhydrique (HF) et une composition de départ comprenant au moins un composé chloré sélectionné parmi le groupe consistant en 1,1,3,3-tetrachloropropene, 1,3,3,3-tetrachloropropene et un mélange des deux, pour obtenir un courant **B** comprenant 1-chloro-3,3,3-trifluoropropène, HF et HCl ;
ii) mise en oeuvre du procédé de purification de 1-chloro-3,3,3-trifluoropropène selon la présente invention à partir d'un courant comprenant 1-chloro-3,3,3-trifluoropropène, HF et/ou HCl.

De préférence, ledit procédé de production du trans-1-chloro-3,3,3-trifluoropropène comprenant les étapes de :
i) mise en contact, dans un réacteur, entre l'acide fluorhydrique (HF) et une composition de départ comprenant au moins un composé chloré sélectionné parmi le groupe consistant en 1,1,3,3-tetrachloropropene, 1,3,3,3-tetrachloropropene et un mélange des deux, pour obtenir un courant **B** comprenant trans-1-chloro-3,3,3-trifluoropropène, HF et HCl ;
ii) mise en oeuvre du procédé de purification de trans-1-chloro-3,3,3-trifluoropropène selon la présente invention à partir d'un courant comprenant trans-1-chloro-3,3,3-trifluoropropène, HF et/ou HCl.

En particulier, ledit procédé de production du trans-1-chloro-3,3,3-trifluoropropène comprenant les étapes de :
i) mise en contact, dans un réacteur, entre l'acide fluorhydrique (HF) et une composition de départ comprenant au moins un composé chloré sélectionné parmi le groupe consistant en 1,1,3,3-tetrachloropropene, 1,3,3,3-tetrachloropropene et un mélange des deux, pour obtenir un courant **B** comprenant trans-1-chloro-3,3,3-trifluoropropène, cis-1-chloro-3,3,3-trifluoropropène, HF et HCl ;
ii) mise en oeuvre du procédé de purification de trans-1-chloro-3,3,3-trifluoropropène selon la présente invention à partir d'un courant comprenant trans-1-chloro-3,3,3-trifluoropropène, cis-1-chloro-3,3,3-trifluoropropène, HF et/ou HCl.

Plus particulièrement, dans le courant **B** et dans le courant utilisé pour la mise en oeuvre de l'étape ii), le ratio molaire entre l'isomère trans et cis du 1-chloro-3,3,3-trifluoropropène est de 2 :1 à 50 :1, de préférence de 5 :1 à 50 :1, en particulier de 9 :1 à 50 :1.

De préférence, ladite étape i) est mise en oeuvre dans une phase liquide, de préférence pauvre en HF.

Selon un mode de réalisation préféré, ladite composition de départ comprend au moins 10% en poids dudit au moins un composé chloré sur base du poids total de ladite composition de départ. Avantageusement, ladite composition de départ comprend au moins 15% en poids dudit au moins un composé chloré, de préférence au moins 20% en poids dudit au moins un composé chloré, plus préférentiellement au moins 25% en poids dudit au moins un composé chloré, en particulier au moins 30% en poids dudit au moins un composé chloré, plus particulièrement au moins 35% en poids dudit au moins un composé chloré, de manière privilégiée au moins 40% en poids dudit au moins un composé chloré, de manière avantageusement privilégiée au moins 45% en poids dudit au moins un composé chloré, de manière préférentiellement privilégiée au moins 50% en poids dudit au moins un composé chloré, de manière particulièrement privilégiée au moins 55% en poids dudit au moins un composé chloré sur base du poids total de ladite composition de départ.

De préférence, ladite composition de départ comprend au moins 60% en poids ou au moins 65% en poids ou au moins 70% en poids ou au moins 75 % en poids ou au moins 80% en poids ou au moins 85% en poids ou au moins 90% en poids ou au moins 95% en poids ou au moins 99% en poids dudit au moins un composé chloré sur base du poids total de ladite composition de départ. Selon un mode de réalisation préféré, ledit au moins un composé chloré est le 1,1,3,3-tétrachloropropène (1230za). Ainsi, ledit procédé comprend une étape i) de mise en contact dans un réacteur entre l'acide fluorhydrique (HF) et une composition de départ comprenant 1,1,3,3-tétrachloropropène (1230za) pour produire un courant **B** comprenant le 1-chloro-3,3,3-trifluoropropène (1233zd), HF et HCl ; ladite étape i) est mise en oeuvre dans une phase liquide pauvre en HF telle que définie ci-dessous.

De préférence, le présent procédé permet la production de 1-chloro-3,3,3-trifluoropropène sous la forme d'un mélange des deux isomères cis et trans. Le présent procédé permet d'obtenir majoritairement l'isomère trans du 1-chloro-3,3,3-trifluoropropène, de préférence le courant **B** comprend au moins 90% en mole de trans-1-chloro-3,3,3-trifluoropropène. En particulier, le courant **B** comprend au moins 95% en mole de trans-1-chloro-3,3,3-trifluoropropène.

Ainsi, ladite composition de départ comprend au moins 10% en poids de 1,1,3,3-tétrachloropropène sur base du poids total de ladite composition de départ. Avantageusement, ladite composition de départ comprend au moins 15% en poids de 1,1,3,3-tétrachloropropène, de préférence au moins 20% en poids de 1,1,3,3-tétrachloropropène, plus préférentiellement au moins 25% en poids de 1,1,3,3-tétrachloropropène, en particulier au moins 30% en poids de 1,1,3,3-tétrachloropropène, plus particulièrement au moins 35% en poids de 1,1,3,3-tétrachloropropène, de manière privilégiée au moins 40% en poids de 1,1,3,3-tétrachloropropène, de manière avantageusement privilégiée au moins 45% en poids de 1,1,3,3-tétrachloropropène, de manière préférentiellement privilégiée au moins 50% en poids de 1,1,3,3-tétrachloropropène, de manière particulièrement privilégiée au moins 55% en poids de 1,1,3,3-tétrachloropropène sur base du poids total de ladite composition de départ.

De préférence, ladite composition de départ comprend au moins 60% en poids ou au moins 65% en poids ou au moins 70% en poids ou au moins 75 % en poids ou au moins 80% en poids ou au moins 85% en poids ou au moins 90% en poids ou au moins 95% en poids ou au moins 99% en poids de 1,1,3,3-tétrachloropropène sur base du poids total de ladite composition de départ. Selon un mode de réalisation préféré, ladite composition de départ comprend moins de 15% en poids de HF sur base du poids total de ladite composition de départ, avantageusement moins de 10% en poids de HF, de préférence moins de 8% en poids de HF, plus préférentiellement moins de 6% en poids de HF, en particulier moins de 5% en poids de HF, plus particulièrement moins de 4% en poids de HF, de manière privilégiée moins de 2% en poids de HF sur base du poids total de ladite composition de départ. Dans le présent procédé, de préférence, la composition de départ est dépourvue de HF. Le terme « dépourvu » signifie une teneur pondérale inférieure à 500 ppm, de préférence inférieure à 100 ppm, en particulier inférieure à 10 ppm.

De préférence, ladite phase liquide pauvre en HF est une phase liquide comprenant moins de 15% en poids de HF, avantageusement moins de 10% en poids de HF, de préférence moins de 8% en poids de HF, plus préférentiellement moins de 6% en poids de HF, en particulier moins de 5% en poids de HF, plus particulièrement moins de 4% en poids de HF, de manière privilégiée moins de 2% en poids de HF sur base du poids total de ladite phase liquide.

Au cours de la mise en oeuvre de l'étape i), ladite phase liquide peut comprendre au moins 10% en poids de composés de formule (I) C₃HₙFₘClₚ (I) dans laquelle n est un entier de 0 à 8, m est un entier de 0 à 8, et p est un entier de 0 à 8 ; de préférence n est un entier de 0 à 8, m est un entier de 0 à 6 et p est un entier de 0 à 6. Par exemple, des composés de formule (I) peuvent être C₃Cl₆, C₃H₄Cl₄ ou C₃H₃Cl₅. De préférence, au cours de la mise en oeuvre de l'étape i), ladite phase liquide peut comprendre au moins 10% en poids de composés de formule (I) C₃HₙFₘClₚ (I) dans laquelle n est un entier de 1 à 8, m est un entier de 0 à 4, et p est un entier de 0 à 4 ; de préférence n est un entier de 1 à 4, m est un entier de 0 à 3 et p est un entier de 2 à 4. Les composés de formule (I) peuvent être des composés de type propane ou propène comprenant un ou plusieurs atomes de chlore et/ou un ou plusieurs atomes de fluor. De préférence, ladite phase liquide peut comprendre au moins 10% en poids de composés de formule (I) sélectionnée parmi le groupe consistant en C₃H₂Cl₄, C₃H₂Cl₃F, C₃H₂Cl₂F₂, C₃H₃Cl₅, C₃H₃Cl₄F, C₃H₃Cl₃F₂ et C₃H₃Cl₂F₃. En particulier, ladite phase liquide peut comprendre au moins 10% en poids de composés de formule (I) sélectionnée parmi le groupe consistant en C₃H₂Cl₄, C₃H₂Cl₃F et C₃H₂Cl₂F₂. Ladite phase liquide peut comprendre au moins 15% en poids de composés de formule (I) C₃HₙFₘClₚ (I) dans laquelle n est un entier de 0 à 8, m est un entier de 0 à 8, et p est un entier de 0 à 8 ; de préférence n est un entier de 0 à 8, m est un entier de 0 à 6 et p est un entier de 0 à 6. En particulier, au cours de la mise en oeuvre de l'étape i), ladite phase liquide peut comprendre au moins 15% en poids de composés de formule (I) C₃HₙFₘClₚ (I) dans laquelle n est un entier de 1 à 8, m est un entier de 0 à 4, et p est un entier de 0 à 4; de préférence n est un entier de 1 à 4, m est un entier de 0 à 3 et p est un entier de 2 à 4. De préférence, ladite phase liquide peut comprendre au moins 15% en poids de composés de formule (I) sélectionnée parmi le groupe consistant en C₃H₂Cl₄, C₃H₂Cl₃F, C₃H₂Cl₂F₂, C₃H₃Cl₅, C₃H₃Cl₄F, C₃H₃Cl₃F₂ et C₃H₃Cl₂F₃. En particulier, ladite phase liquide peut comprendre au moins 15% en poids de composés de formule (I) sélectionnée parmi le groupe consistant en C₃H₂Cl₄, C₃H₂Cl₃F et C₃H₂Cl₂F₂. Ladite phase liquide peut comprendre au moins 20%, au moins 30%, au moins 40%, au moins 50%, au moins 60%, au moins 70%, au moins 80% ou au moins 90% en poids de composés de formule (I) C₃HₙFₘClₚ (I) dans laquelle n est un entier de 0 à 8, m est un entier de 0 à 8, et p est un entier de 0 à 8 ; de préférence n est un entier de 0 à 8, m est un entier de 0 à 6 et p est un entier de 0 à 6. Ladite phase liquide peut comprendre au moins 20%, au moins 30%, au moins 40%, au moins 50%, au moins 60%, au moins 70%, au moins 80% ou au moins 90% en poids de composés de formule (I) C₃HₙFₘClₚ (I) dans laquelle n est un entier de 1 à 8, m est un entier de 0 à 4, et p est un entier de 0 à 4; de préférence n est un entier de 1 à 4, m est un entier de 0 à 3 et p est un entier de 2 à 4. De préférence, ladite phase liquide peut comprendre au moins 20%, au moins 30%, au moins 40%, au moins 50%, au moins 60%, au moins 70%, au moins 80% ou au moins 90% en poids de composés de formule (I) sélectionnée parmi le groupe consistant en C₃H₂Cl₄, C₃H₂Cl₃F, C₃H₂Cl₂F₂, C₃H₃Cl₅, C₃H₃Cl₄F, C₃H₃Cl₃F₂ et C₃H₃Cl₂F₃. En particulier, ladite phase liquide peut comprendre au moins 20%, au moins 30%, au moins 40%, au moins 50%, au moins 60%, au moins 70%, au moins 80% ou au moins 90% en poids de composés de formule (I) sélectionnée parmi le groupe consistant en C₃H₂Cl₄, C₃H₂Cl₃F et C₃H₂Cl₂F₂.

De préférence, l'étape i) est mise en oeuvre en l'absence de catalyseur.

Alternativement, l'étape i) peut être mise en oeuvre en présence d'un catalyseur. Le catalyseur peut être un catalyseur de type TiCl₄ ou SbCl₅. Le catalyseur peut être également un liquide ionique. Les liquides ioniques pouvant convenir sont dérivés d'acides de Lewis à base d'aluminium, de titane, de niobium, de tantale, d'étain, d'antimoine, de nickel, de zinc ou de fer. On entend par liquides ioniques des sels non aqueux à caractère ionique qui sont liquides à des températures modérées (de préférence en-dessous de 120°C). Les liquides ioniques résultent de préférence de la réaction entre un sel organique et un composé inorganique. Les liquides ioniques sont de préférence obtenus par réaction d'au moins un acide de Lewis halogéné ou oxyhalogéné à base d'aluminium, de titane, de niobium, de tantale, d'étain, d'antimoine, de nickel, de zinc ou de fer avec un sel de formule générale Y⁺A⁻, dans laquelle A⁻ désigne un anion halogénure (bromure, iodure et, de préférence chlorure ou fluorure) ou hexafluoroantimoniate (SbF₆⁻) et Y⁺ un cation ammonium quaternaire, phosphonium quaternaire ou sulfonium ternaire. L'acide de Lewis halogéné à base d'aluminium, de titane, de niobium, de tantale, d'antimoine, de nickel, de zinc ou de fer peut être un dérivé chloré, bromé, fluoré ou mixte, par exemple un acide chlorofluoré. Peuvent être mentionnés plus particulièrement les chlorures, fluorures ou chlorofluorures des formules suivantes :
TiClₓF_{y} avec x+y = 4 et 0 <= x <= 4
TaClₓF_{y} avec x+y = 5 et 0 <= x <= 5
NbClₓF_{y} avec x+y = 5 et 0 <= x <= 5
SnClₓF_{y} avec x+y = 4 et 1 <= x <= 4
SbClₓF_{y} avec x+y = 5 et 0 <= x <= 5
AlClₓF_{y} avec x+y =3 et 0 <= x <= 3
NiClₓF_{y} avec x+y = 2 et 0 <= x <= 2
FeClₓF_{y} avec x+y = 3 et 0 <= x <= 3

Comme exemples de tels composés, on peut mentionner les composés suivants : TiCl₄, TiF₄, TaCl₅, TaF₅, NbCl₅, NbF₅, SbCl₅, SbCl₄F, SbCl₃F₂, SbCl₂F₃, SbClF₄, SbF₅ et leurs mélanges. Sont préferentiellement utilisés les composés suivants : TiCl₄, TaCl₅+TaF₅, NbCl₅+NbF₅, SbCl₅, SbFCl₄, SbF₂Cl₃, SbF₃Cl₂, SbF₄Cl, SbF₅ et SbCl₅+SbF₅. Sont plus particulièrement préférés les composés antimoniés. Comme exemples d'acides de Lewis oxyhalogénés, utilisables selon l'invention, on peut mentionner TiOCl₂, TiOF₂ et SbOClₓF_{y} (x+y=3). Dans le sel Y⁺A⁻, le cation Y⁺ peut répondre à l'une des formules générales suivantes: R¹R²R³R⁴N⁺, R¹R²R³R⁴P⁺, R¹R²R³S⁺ dans lesquelles les symboles R¹ a R⁴, identiques ou différents, désignent chacun un groupement hydrocarbyle, chlorohydrocarbyle, fluorohydrocarbyle, chlorofluorohydrocarbyle ou fluorocarbyle ayant de 1 a 10 atomes de carbone, saturé ou non, cyclique ou non, ou aromatique, l'un ou plusieurs de ces groupements pouvant également contenir un ou plusieurs hétéroatomes tels que N, P, S ou O. Le cation ammonium, phosphonium ou sulfonium Y⁺ peut également faire partie d'un hétérocycle saturé ou non, ou aromatique ayant de 1 à 3 atomes d'azote, de phosphore ou de soufre, et répondre à l'une ou l'autre des formules générales suivantes : 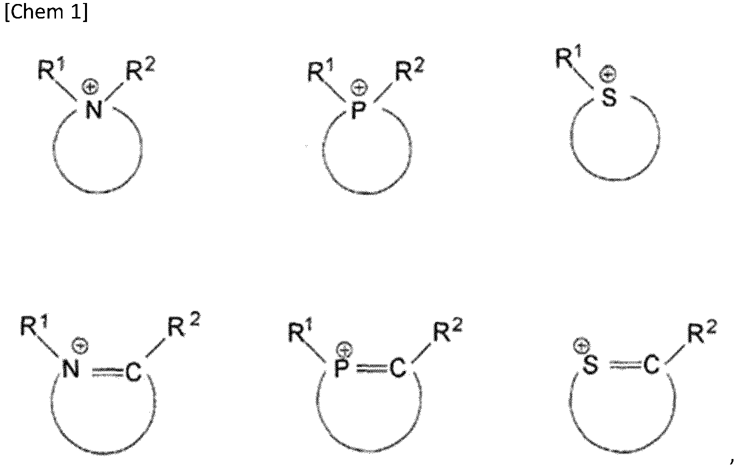 dans lesquelles R¹ et R² sont tels que définis précédemment. Un sel contenant 2 ou 3 sites ammonium, phosphonium ou sulfonium dans leur formule peut également convenir. Comme exemples de sels Y⁺A⁻ on peut mentionner les chlorures et fluorures de tétraalkyl ammonium, les chlorures et fluorures de tétraalkyl phosphonium, et chlorures et fluorures de trialkyl sulfonium, les chlorures et fluorures d'alkyl pyridinium, les chlorures, fluorures et bromures de dialkyl imidazolium, et les chlorures et fluorures de trialkyl imidazolium. Sont plus particulièrement appréciés le fluorure ou le chlorure de trimethyl sulfonium, le chlorure ou le fluorure de N-ethyl-pyridinium, le chlorure ou le fluorure de N-butyl-pyridinium, le chlorure ou le fluorure de 1-ethyl-3-methyl-imidazolium, et le chlorure ou fluorure de 1-butyl-3-methyl-imidazolium. Les liquides ioniques peuvent être préparés de façon connue en soi en mélangeant de manière appropriée l'acide de Lewis halogéné ou oxyhalogéné et le sel organique Y⁺A⁻. On peut se reporter notamment à la méthode décrite dans le document WO 01/81353. Alternativement, le catalyseur peut être l'acide triflique ou trifluoroacétique tel que mentionné dans le document US 6,166,274.

Selon un mode de réalisation préféré, outre le trans/cis-1-chloro-3,3,3-trifluoropropène, le courant B comprend des coproduits sélectionnés parmi le groupe consistant en 1,3,3,3-tétrafluoropropène et 1,1,1,3,3-pentafluoropropane. Selon un mode de réalisation préféré, la teneur dans la courant B en coproduits sélectionnés parmi le groupe consistant en 1,3,3,3-tétrafluoropropène et 1,1,1,3,3-pentafluoropropane est inférieure à 0,5 % en mole. De préférence, la teneur en 1,3,3,3-tétrafluoropropène dans ledit courant **B** est inférieure à 0,5%, plus préférentiellement inférieure à 0,4%, en particulier inférieure à 0,3% en mole. De préférence, la teneur en 1,1,1,3,3-pentafluoropropane dans ledit courant **B** est inférieure à 0,1%, plus préférentiellement inférieure à 0,075%, en particulier inférieure à 0,05% en mole. De préférence, l'étape i) est mise en oeuvre à une température de 50°C à 150°C, de préférence à une température de 75°C à 100°C.

De préférence, l'étape i) est mise en oeuvre à une pression de 5 à 20 bara, de préférence à une pression de 10 à 18 bara, en particulier de 12 à 18 bara.

De préférence, le ratio molaire HF/[composés chlorés] à l'entrée du réacteur est compris entre 5 et 10, plus préférentiellement entre 5 et 7, en particulier entre 5 et 6. En particulier, lorsque ledit composé chloré de la composition de départ est 1,1,3,3-tétrachloropropène (1230za), le ratio molaire HF/1230za est compris entre 5 et 10, plus préférentiellement entre 5 et 7, en particulier entre 5 et 6.

L'acide fluorhydrique et ladite composition de départ peuvent être introduits dans le réacteur par l'intermédiaire d'un mélangeur statique. De préférence, l'acide fluorhydrique est chauffé avant son introduction dans le réacteur et ainsi avant la mise en oeuvre de l'étape i). De préférence, l'acide fluorhydrique est chauffé à une température de 100°C à 170°C, de préférence de 120°C à 170°C, en particulier de 125°C à 165°C, plus particulièrement de 125°C à 155°C. Selon un mode de réalisation particulier, le courant **B** obtenu à l'étape i) peut être soumis à des étapes de purification préalablement à la mise en oeuvre de l'étape ii). Alternativement, le courant **B** obtenu à l'étape i) peut être utilisé directement à l'étape ii).

Ainsi, ledit procédé de production peut comprendre une étape i'), subséquente à l'étape i) et préalable à l'étape ii), comprenant une étape de traitement du courant **B** pour donner un courant **B1** comprenant le 1-chloro-3,3,3-trifluoropropène, HCl, HF et un courant **B2** comprenant au moins 50% en poids d'HF, par exemple de préférence au moins 70% en poids d'HF. L'étape de traitement (i') est de préférence une colonne de reflux, mise en oeuvre avantageusement à une température comprise entre 30 et 120°C pour donner le courant **B2** qui est recyclé au réacteur. De préférence, dans le courant **B1,** le ratio molaire entre l'isomère trans et cis du 1-chloro-3,3,3-trifluoropropène est de 2 :1 à 50 :1, de préférence de 5 :1 à 50 :1, en particulier de 9 :1 à 50 :1.

Selon un mode de réalisation particulier, ledit procédé de production du 1-chloro-3,3,3-trifluoropropène selon la présente invention comprenant les étapes de :
i) mise en contact, dans un réacteur, entre l'acide fluorhydrique (HF) et une composition de départ comprenant au moins un composé chloré sélectionné parmi le groupe consistant en 1,1,3,3-tetrachloropropene, 1,3,3,3-tetrachloropropene et un mélange des deux, pour obtenir un courant **B** comprenant 1-chloro-3,3,3-trifluoropropène, HF et HCl ;
i') une étape de traitement du courant **B** pour donner un courant **B1** comprenant le 1-chloro-3,3,3-trifluoropropène, HCl, HF et un courant **B2** comprenant au moins 50% en poids d'HF ;
ii) mise en oeuvre du procédé de purification de 1-chloro-3,3,3-trifluoropropène selon la présente invention à partir du courant **B1** obtenu à l'étape i').

Le présent procédé de production peut également comprendre une étape ii') subséquente à l'étape i') et préalable à l'étape ii). De préférence, ladite étape ii') est une étape de récupération de l'acide chlorhydrique du courant **B1** pour former un courant **B3** d'HCl et un courant **B4** comprenant le 1-chloro-3,3,3-trifluoropropène, HCl, HF. La récupération d'HCl à l'étape (ii') est de préférence obtenue à l'aide d'une colonne de distillation munie d'un rebouilleur en pied et d'un système de reflux en tête. La température en pied est avantageusement comprise entre 20 et 110°C. La température en tête est avantageusement comprise entre -50 et 0°C. La distillation de l'HCl est typiquement effectuée à une pression comprise entre 7 et 25 bars. Cette étape de récupération permet d'obtenir un courant **B4** dans lequel la quantité d'HCl est fortement diminuée par rapport à la quantité d'HCl dans le courant **B1.**

Ainsi, selon un mode de réalisation particulier, ledit procédé de production du 1-chloro-3,3,3-trifluoropropène selon la présente invention comprenant les étapes de :
i) mise en contact, dans un réacteur, entre l'acide fluorhydrique (HF) et une composition de départ comprenant au moins un composé chloré sélectionné parmi le groupe consistant en 1,1,3,3-tetrachloropropene, 1,3,3,3-tetrachloropropene et un mélange des deux, pour obtenir un courant **B** comprenant 1-chloro-3,3,3-trifluoropropène, HF et HCl ;
i') traitement du courant **B** pour donner un courant **B1** comprenant le 1-chloro-3,3,3-trifluoropropène, HCl, HF et un courant **B2** comprenant au moins 50% en poids d'HF ;
ii') récupération de l'acide chlorhydrique du courant **B1** pour former un courant **B3** d'HCl et un courant **B4** comprenant le 1-chloro-3,3,3-trifluoropropène, HCl, HF ;
ii) mise en oeuvre du procédé de purification de 1-chloro-3,3,3-trifluoropropène selon la présente invention à partir du courant **B4** obtenu à l'étape ii') pour former le courant neutralisé **A1.** De préférence, dans le courant **B4,** le ratio molaire entre l'isomère trans et cis du 1-chloro-3,3,3-trifluoropropène est de 2 :1 à 50 :1, de préférence de 5 :1 à 50 :1, en particulier de 9 :1 à 50 :1.

Le présent procédé de production peut également comprendre une étape iii') subséquente à l'étape ii') et préalable à l'étape ii). De préférence, l'étape iii') est une étape de séparation pour former un courant **B5** comprenant au moins 90% en poids, de préférence au moins 98% en poids et en particulier au moins 99% en poids d'HF, et un courant **B6** comprenant 1-chloro-3,3,3-trifluoropropène, HCl et HF. L'étape de séparation est de préférence une décantation, mise en oeuvre à une température avantageusement comprise entre -50 et 50°C, de préférence entre - 20°C et 10°C.

Ainsi, selon un mode de réalisation particulier, ledit procédé de production du 1-chloro-3,3,3-trifluoropropène selon la présente invention comprenant les étapes de :
i) mise en contact, dans un réacteur, entre l'acide fluorhydrique (HF) et une composition de départ comprenant au moins un composé chloré sélectionné parmi le groupe consistant en 1,1,3,3-tetrachloropropene, 1,3,3,3-tetrachloropropene et un mélange des deux, pour obtenir un courant **B** comprenant 1-chloro-3,3,3-trifluoropropène, HF et HCl ;
i') traitement du courant **B** pour donner un courant **B1** comprenant le 1-chloro-3,3,3-trifluoropropène, HCl, HF et un courant **B2** comprenant au moins 50% en poids d'HF ;
ii') récupération de l'acide chlorhydrique du courant **B1** pour former un courant **B3** d'HCl et un courant **B4** comprenant le 1-chloro-3,3,3-trifluoropropène, HCl, HF ;
iii') séparation du courant **B4** obtenu à l'étape ii') pour former un courant **B5** comprenant au moins 90% en poids et un courant **B6** comprenant 1-chloro-3,3,3-trifluoropropène, HCl et HF ;
ii) mise en oeuvre du procédé de purification de 1-chloro-3,3,3-trifluoropropène selon la présente invention à partir du courant **B6** obtenu à l'étape iii') pour former le courant neutralisé **A1.** De préférence, dans le courant **B6,** le ratio molaire entre l'isomère trans et cis du 1-chloro-3,3,3-trifluoropropène est de 2 :1 à 50 :1, de préférence de 5 :1 à 50 :1, en particulier de 9 :1 à 50 :1.

Le présent procédé de production peut également comprendre une étape iv') subséquente à l'étape iii') et préalable à l'étape ii). De préférence, l'étape iv') est une étape de lavage à l'eau. Cette étape permet éliminer une partie du HCl et du HF compris dans le courant **B6.**

Ainsi, selon un mode de réalisation particulier, ledit procédé de production du 1-chloro-3,3,3-trifluoropropène selon la présente invention comprenant les étapes de :
i) mise en contact, dans un réacteur, entre l'acide fluorhydrique (HF) et une composition de départ comprenant au moins un composé chloré sélectionné parmi le groupe consistant en 1,1,3,3-tetrachloropropene, 1,3,3,3-tetrachloropropene et un mélange des deux, pour obtenir un courant **B** comprenant 1-chloro-3,3,3-trifluoropropène, HF et HCl ;
i') traitement du courant **B** pour donner un courant **B1** comprenant le 1-chloro-3,3,3-trifluoropropène, HCl, HF et un courant **B2** comprenant au moins 50% en poids d'HF ;
ii') récupération de l'acide chlorhydrique du courant **B1** pour former un courant **B3** d'HCl et un courant **B4** comprenant le 1-chloro-3,3,3-trifluoropropène, HCl, HF ;
iii') séparation du courant **B4** obtenu à l'étape ii') pour former un courant **B5** comprenant au moins 90% en poids et un courant **B6** comprenant 1-chloro-3,3,3-trifluoropropène, HCl et HF ;
iv') lavage dudit courant **B6** obtenu à l'étape iii') avec de l'eau pour former un courant **B7** comprenant 1-chloro-3,3,3-trifluoropropène et une solution aqueuse acide **B8** ;
ii) mise en oeuvre du procédé de purification de 1-chloro-3,3,3-trifluoropropène selon la présente invention à partir du courant **B7** obtenu à l'étape iv') pour former le courant neutralisé **A1.** De préférence, dans le courant **B7,** le ratio molaire entre l'isomère trans et cis du 1-chloro-3,3,3-trifluoropropène est de 2 :1 à 50 :1, de préférence de 5 :1 à 50 :1, en particulier de 9 :1 à 50 :1.

Le présent procédé de production peut également comprendre une étape iii) subséquente à l'étape ii). De préférence, l'étape iii) consiste en un séchage dudit courant neutralisé **A1** comprenant 1-chloro-3,3,3-trifluoropropène pour former un courant séché **A2** comprenant 1-chloro-3,3,3-trifluoropropène. L'étape de séchage peut être effectuée à l'aide de tamis moléculaire, de zéolithe, de sels inorganiques tels que le sulfate de calcium ou le chlorure de calcium, de gel de silice, de charbon actif. Des exemples de tamis moléculaires et de zéolithes sont décrits dans le document WO2017/050686. Des exemples de sels inorganiques, de tamis moléculaires, de gel de silice et de charbon actif sont également décrits dans WO2017031406. Des exemples de tamis moléculaires, de charbon actif et de gel de silice sont également décrits dans WO2016/148957. De préférence, l'étape de séchage s'effectuera à l'aide de tamis moléculaire, en particulier à l'aide d'un tamis moléculaire 3A.

Le présent procédé de production peut également comprendre une étape iv) subséquente à l'étape iii). De préférence, l'étape iv) comprend une ou plusieurs étapes de distillation dudit courant **A2** obtenu à l'étape iii).

Ainsi, selon un mode de réalisation particulier, ledit procédé de production du 1-chloro-3,3,3-trifluoropropène selon la présente invention comprenant les étapes de :
i) mise en contact, dans un réacteur, entre l'acide fluorhydrique (HF) et une composition de départ comprenant au moins un composé chloré sélectionné parmi le groupe consistant en 1,1,3,3-tetrachloropropene, 1,3,3,3-tetrachloropropene et un mélange des deux, pour obtenir un courant **B** comprenant 1-chloro-3,3,3-trifluoropropène, HF et HCl ;
i') traitement du courant **B** pour donner un courant **B1** comprenant le 1-chloro-3,3,3-trifluoropropène, HCl, HF et un courant **B2** comprenant au moins 50% en poids d'HF ;
ii') récupération de l'acide chlorhydrique du courant **B1** pour former un courant **B3** d'HCl et un courant **B4** comprenant le 1-chloro-3,3,3-trifluoropropène, HCl, HF ;
iii') séparation du courant **B4** obtenu à l'étape ii') pour former un courant **B5** comprenant au moins 90% en poids et un courant **B6** comprenant 1-chloro-3,3,3-trifluoropropène, HCl et HF ;
iv') lavage dudit courant **B6** obtenu à l'étape iii') avec de l'eau pour former un courant **B7** comprenant 1-chloro-3,3,3-trifluoropropène et une solution aqueuse acide **B8** ;
ii) mise en oeuvre du procédé de purification de 1-chloro-3,3,3-trifluoropropène selon la présente invention à partir du courant **B7** obtenu à l'étape iv') pour former le courant neutralisé **A1;**
iii) séchage dudit courant neutralisé **A1** comprenant 1-chloro-3,3,3-trifluoropropène pour former un courant séché **A2** comprenant 1-chloro-3,3,3-trifluoropropène ; et
iv) distillation dudit courant **A2** à l'aide d'une ou plusieurs colonnes de distillation pour former un courant **A3** comprenant 1-chloro-3,3,3-trifluoropropène.

De préférence, dans le courant **B1, B4, B6** et **B7,** le ratio molaire entre l'isomère trans et cis du 1-chloro-3,3,3-trifluoropropène est de 2 :1 à 50 :1, de préférence de 5 :1 à 50 :1, en particulier de 9 :1 à 50 :1.

De préférence, ledit procédé de production selon la présente invention est mis en oeuvre en continu.

### Exemples

Les exemples ci-dessous visent à étudier le comportement d'un flux comprenant notamment du trans-1-chloro-3,3,3-trifluoropropène en présence de différentes solutions basiques. Les exemples sont mis en oeuvre dans un réacteur surmonté d'une colonne d'abattage (tous les deux étant calorifugés). La colonne d'abattage (diamètre intérieur = 24 mm, hauteur = 300 mm) est garnie avec des anneaux de Raschig (diamètre intérieur 3 mm, diamètre extérieur = 5 mm, longueur = 5 mm, fraction de vie dans la colonne garnie = 63%). On a introduit une solution basique et un tourne-en-rond de cette solution est mise en oeuvre à travers une colonne d'abattage garnie avec des anneaux de Raschig (environ 185 ml/min). La solution basique est portée à une température de 30°C. Un courant comprenant 1-chloro-3,3,3-trifluoropropène (95,5% en mole de l'isomère trans et 2,5% en mole de l'isomère cis) a ensuite été introduit dans le réacteur à un débit de 5 g/h. Le flux gazeux a été récupéré après neutralisation pour être séché sur du CaCl₂ anhydre et piégé à l'aide d'un piège à l'azote liquide. Le flux gazeux a été analysé par chromatographie phase gazeuse. On détermine la quantité de trifluoropropyne formée après neutralisation pour évaluer l'impact des solutions basiques testées sur le 1-chloro-3,3,3-trifluoropropène. Trois solutions basiques ont été testées et les résultats sont repris ci-dessous dans le tableau 1.

**[Tableau 1]**

| Exemple | Solution basique | Teneur en trifluoropropyne |
|---|---|---|
| Exemple 1 (Comp.) | 5% KOH | 3840 ppm |
| Exemple 2 (Comp.) | 5% NaOH | 2410 ppm |
| Exemple 3 (Inv.) | 5% NaOH + 5% Na₂SO₃ | 1830 ppm |

Comme démontré par les exemples ci-dessus, l'utilisation d'une solution basique comprenant un sel de sulfite et un hydroxyde alcalin permet de limiter la formation de trifluoropropyne lors de l'étape de neutralisation. Le procédé selon la présente invention est par conséquent plus efficace qu'un procédé mettant en oeuvre une étape de neutralisation en présence de KOH ou NaOH seul.

## Revendications

1. Procédé de purification du 1-chloro-3,3,3-trifluoropropène comprenant les étapes de :
a) fourniture d'un courant comprenant 1-chloro-3,3,3-trifluoropropène et au moins un composé de formule HX dans laquelle X est F ou CI ;
b) mise en contact du courant de l'étape a) avec une solution **A** comprenant au moins un hydroxyde alcalin ou un hydroxyde d'alcalino-terreux et au moins un sel de sulfite de formule Yⁿ⁺ₘSO₃ dans laquelle Y est un métal alcalin ou alcalino-terreux, n = 1 ou 2, et m = 2 lorsque n = 1 ou m = 1 lorsque n = 2 ; pour former un courant neutralisé **A1** comprenant 1-chloro-3,3,3-trifluoropropène.

2. Procédé selon la revendication précédente **caractérisé en ce que** ledit au moins un sel de sulfite est sélectionné parmi le groupe consistant en Na₂SO₃, K₂SO₃, Li₂SO₃, CaSO₃ et MgSO₃ ou un mélange de ceux-ci.

3. Procédé selon l'une quelconque des revendications précédentes **caractérisé en ce que** ledit au moins un sel de sulfite est Na₂SO₃ ou K₂SO₃ ou un mélange des deux.

4. Procédé selon l'une quelconque des revendications précédentes **caractérisé en ce que** ladite solution **A** comprend au moins un hydroxyde alcalin, de préférence NaOH.

5. Procédé selon l'une quelconque des revendications précédentes **caractérisé en ce que** ladite solution **A** de l'étape b) comprend NaOH et Na₂SO₃.

6. Procédé selon l'une quelconque des revendications précédentes **caractérisé en ce que** ladite solution **A** de l'étape b) est une solution aqueuse.

7. Procédé selon l'une quelconque des revendications précédentes **caractérisé en ce que** la teneur en ledit au moins un sel de sulfite est comprise entre 2 et 25% en poids sur base du poids total de ladite solution **A.**

8. Procédé selon l'une quelconque des revendications précédentes **caractérisé en ce que** l'étape b) est mise en oeuvre à une température de 10°C à 70°C.

9. Procédé selon l'une quelconque des revendications précédentes **caractérisé en ce que** la teneur en hydroxyde alcalin ou en hydroxyde d'alcalino-terreux est comprise entre 2 et 25% en poids sur base du poids total de ladite solution **A.**

10. Procédé selon l'une quelconque des revendications précédentes **caractérisé en ce que** l'étape b) est mise en oeuvre avec une solution aqueuse **A** comprenant de 3 à 10% de Na₂SO₃, de 3 à 10% de NaOH ; et à une température de 20°C à 60°C

11. Procédé selon l'une quelconque des revendications précédentes **caractérisé en ce que** dans le courant fourni à l'étape a), le ratio molaire entre l'isomère trans et l'isomère cis du 1-chloro-3,3,3-trifluoropropène est de 2 :1 à 50 :1, de préférence de 5 :1 à 50 :1, en particulier de 9 :1 à 50 :1.

12. Procédé de production du 1-chloro-3,3,3-trifluoropropène comprenant les étapes de :
i) mise en contact, dans un réacteur, entre l'acide fluorhydrique (HF) et une composition de départ comprenant au moins un composé chloré sélectionné parmi le groupe consistant en 1,1,3,3-tetrachloropropene, 1,3,3,3-tetrachloropropene et un mélange des deux, pour obtenir un courant **B** comprenant 1-chloro-3,3,3-trifluoropropène, HF et/ou HCl ;
ii) mise en oeuvre du procédé de purification de 1-chloro-3,3,3-trifluoropropène selon l'une quelconque des revendications précédentes à partir d'un courant comprenant 1-chloro-3,3,3-trifluoropropène, HF et/ou HCl.

## Patentansprüche

1. Verfahren zur Reinigung von 1-Chlor-3,3,3-trifluorpropen, das folgende Schritte umfasst:
a) Bereitstellen eines Stroms, der 1-Chlor-3,3,3-trifluorpropen und mindestens eine Verbindung der Formel HX, in der X für F oder Cl steht, umfasst;
b) Inkontaktbringen des Stroms aus Schritt a) mit einer Lösung **A,** die mindestens ein Alkalihydroxid oder ein Erdalkalihydroxid und mindestens ein Sulfitsalz der Formel Yⁿ⁺ₘSO₃, in der Y für ein Alkali- oder Erdalkalimetall steht, n = 1 oder 2 und m = 2, wenn n = 1, oder m = 1, wenn n = 2, umfasst; zur Bildung eines neutralisierenden Stroms **A1,** der 1-Chlor-3,3,3-trifluorpropen umfasst.

2. Verfahren nach dem vorhergehenden Anspruch, **dadurch gekennzeichnet, dass** das mindestens eine Sulfitsalz aus der Gruppe bestehend aus Na₂SO₃, K₂SO₃, Li₂SO₃, CaSO₃ und MgSO₃ und einer Mischung davon ausgewählt wird.

3. Verfahren nach einem der vorhergehenden Ansprüche, **dadurch gekennzeichnet, dass** es sich bei dem mindestens einen Sulfitsalz um Na₂SO₃ oder K₂SO₃ oder eine Mischung davon handelt.

4. Verfahren nach einem der vorhergehenden Ansprüche, **dadurch gekennzeichnet, dass** die Lösung **A** mindestens ein Alkalihydroxid, vorzugsweise NaOH, umfasst.

5. Verfahren nach einem der vorhergehenden Ansprüche, **dadurch gekennzeichnet, dass** die Lösung **A** von Schritt b) NaOH und Na₂SO₃ umfasst.

6. Verfahren nach einem der vorhergehenden Ansprüche, **dadurch gekennzeichnet, dass** es sich bei der Lösung **A** von Schritt b) um eine wässrige Lösung handelt.

7. Verfahren nach einem der vorhergehenden Ansprüche, **dadurch gekennzeichnet, dass** der Gehalt des mindestens einen Sulfitsalzes zwischen 2 und 25 Gew.-%, bezogen auf das Gesamtgewicht der Lösung **A,** liegt.

8. Verfahren nach einem der vorhergehenden Ansprüche, **dadurch gekennzeichnet, dass** der Schritt b) bei einer Temperatur von 10 °C bis 70 °C durchgeführt wird.

9. Verfahren nach einem der vorhergehenden Ansprüche, **dadurch gekennzeichnet, dass** der Gehalt an Alkalihydroxid oder an Erdalkalihydroxid zwischen 2 und 25 Gew.-%, bezogen auf das Gesamtgewicht der Lösung **A,** liegt.

10. Verfahren nach einem der vorhergehenden Ansprüche, **dadurch gekennzeichnet, dass** der Schritt b) mit einer wässrigen Lösung A, die 3 bis 10 % Na₂SO₃ und 3 bis 10 % NaOH umfasst, und bei einer Temperatur von 20 °C bis 60 °C durchgeführt wird.

11. Verfahren nach einem der vorhergehenden Ansprüche, **dadurch gekennzeichnet, dass** in dem in Schritt a) bereitgestellten Strom das Molverhältnis zwischen dem trans-Isomer und dem cis-Isomer von 1-Chlor-3,3,3-trifluorpropen 2:1 bis 50:1, vorzugsweise 5:1 bis 50:1, insbesondere 9:1 bis 50:1, beträgt.

12. Verfahren zur Herstellung von 1-Chlor-3,3,3-trifluorpropen, das folgende Schritte umfasst:
i) Inkontaktbringen von Fluorwasserstoffsäure (HF) mit einer Ausgangszusammensetzung, die mindestens eine Chlorverbindung aus der Gruppe bestehend aus 1,1,3,3-Tetrachlorpropen, 1,3,3,3-Tetrachlorpropen und einer Mischung davon umfasst, in einem Reaktor zum Erhalt eines Stroms **B,** der 1-Chlor-3,3,3-trifluorpropen, HF und/oder HCl umfasst;
ii) Durchführen des Verfahrens zur Reinigung von 1-Chlor-3,3,3-trifluorpropen nach einem der vorhergehenden Ansprüche ausgehend von einem Strom, der 1-Chlor-3,3,3-trifluorpropen, HF und/oder HCl umfasst.

## Claims

1. Process for purifying 1-chloro-3,3,3-trifluoropropene comprising the steps of:
a) providing a stream comprising 1-chloro-3,3,3-trifluoropropene and at least one compound of formula HX wherein X is F or Cl;
b) bringing the stream from step a) into contact with a solution **A** comprising at least one alkali metal hydroxide or one alkaline-earth metal hydroxide and at least one sulfite salt of formula Yⁿ⁺ₘSO₃ wherein Y is an alkali or alkaline-earth metal, n = 1 or 2, and m = 2 when n = 1 or m = 1 when n = 2; in order to form a neutralized stream **A1** comprising 1-chloro-3,3,3-trifluoropropene.

2. Process according to the preceding claim, **characterized in that** said at least one sulfite salt is selected from the group consisting of Na₂SO₃, K₂SO₃, Li₂SO₃, CaSO₃ and MgSO₃ or a mixture thereof.

3. Process according to either one of the preceding claims, **characterized in that** said at least one sulfite salt is Na₂SO₃ or K₂SO₃ or a mixture of both.

4. Process according to any one of the preceding claims, **characterized in that** said solution **A** comprises at least one alkali metal hydroxide, preferably NaOH.

5. Process according to any one of the preceding claims, **characterized in that** said solution **A** of step b) comprises NaOH and Na₂SO₃.

6. Process according to any one of the preceding claims, **characterized in that** said solution **A** of step b) is an aqueous solution.

7. Process according to any one of the preceding claims, **characterized in that** the content of said at least one sulfite salt is between 2 and 25% by weight based on the total weight of said solution **A.**

8. Process according to any one of the preceding claims, **characterized in that** step b) is carried out at a temperature of 10°C to 70°C.

9. Process according to any one of the preceding claims, **characterized in that** the content of alkali metal hydroxide or alkaline-earth metal hydroxide is between 2 and 25% by weight based on the total weight of said solution **A.**

10. Process according to any one of the preceding claims, **characterized in that** step b) is carried out with an aqueous solution **A** comprising from 3 to 10% of Na₂SO₃ and from 3 to 10% of NaOH; and at a temperature of from 20°C to 60°C.

11. Process according to any one of the preceding claims, **characterized in that**, in the stream provided in step a), the molar ratio between the trans isomer and the cis isomer of 1-chloro-3,3,3-trifluoropropene is from 2:1 to 50:1, preferably 5:1 to 50:1, in particular 9:1 to 50:1.

12. Process for producing 1-chloro-3,3,3-trifluoropropene comprising the steps of:
i) bringing the hydrofluoric acid (HF) into contact, in a reactor, with a starting composition comprising at least one chlorinated compound selected from the group consisting of 1,1,3,3-tetrachloropropene, 1,3,3,3-tetrachloropropene and a mixture of the two, to obtain a stream **B** comprising 1-chloro-3,3,3-trifluoropropene, HF and/or HCl;
ii) carrying out the process for purifying 1-chloro-3,3,3-trifluoropropene according to any one of the preceding claims using a stream comprising 1-chloro-3,3,3-trifluoropropene, HF and/or HCl.
